# EUROPEAN PATENT APPLICATION

(11) **EP 4 813 410 A1**
(43) Date of publication of application: **30.09.2026**
(21) Application number: 25166684.8
(22) Date of filing: 27.03.2025
(51) Int. Cl.: A61L 27/10, A61L 27/12

(54) **AN AQUEOUS GLASS EXTRACT SOLUTION, A METHOD FOR ITS PREPARATION AND ITS USES**

(71) Applicant: Bonalive Oy, 20750 Turku (FI)
(72) Inventor: LUCCHESI, James, 20750 Turku (FI)
(74) Representative: Laine IP Oy

(57) **Abstract**

The present invention relates to a method for manufacturing an aqueous glass extract solution comprising 250-15000 mg/l of Na-ions, 5-300 mg/l of Ca-ions, 1-300 mg/l of P-ions and 30-1500 mg/l of Si-ions, the method comprising obtaining a bioactive glass composition comprising 44-65 weight-% of SiO₂, 5-26 weight-% of Na₂O, 0.5-25 weight-% of CaO and 0.1-7 weight-% of P₂O₅, in the form of particles having a particle size of 1-1000 µm. The method further comprises adding the bioactive glass particles in water or saline solution, in an amount of 1-50 wt-%, extracting the ions into water or the saline solution by allowing the bioactive glass particles to dissolve for an extraction time of 1-120 h at a temperature of at least 20 °C and recovering the supernatant aqueous glass extract solution. The invention also relates to the thus obtained extract solution and its uses.

## Description

### FIELD OF THE INVENTION

The present invention relates to an aqueous glass extract solution as well as to a method for preparing it. The invention also relates to uses of the aqueous glass extract solution.

### BACKGROUND OF THE INVENTION

Bioactive materials can initiate a particular biological reaction between the material and tissue, fostering the formation of a bond and triggering a response within the biological system. Bioactive glasses represent one form of these materials and are specific glass formulations that are capable of reacting with physiological fluids to form strong bonds with bone, helping in bone regeneration and tissue repair. Indeed, it has been observed that bioactive glass can influence aspects such as hemostasis, epithelial cell migration, fibroblast proliferation, stem cell proliferation and angiogenesis.

Bioactive glasses have been used in the medical field for some years. Solid glass is however not suitable for all uses, and for some applications, a product in another form than solid would be preferable.

In particular, various infections of the skin, nails, deep wounds or surgical sites cannot be easily treated with solid glass. For example, chronic wounds like those in diabetic patients with neuropathy are challenging to treat. Diabetic neuropathy can cause nerve and blood vessel damage, leading to a range of symptoms like loss of sensation, pain, foot complications, foot ulcers, balance issues, chronic pain, and problems with organ function. In all these applications, it would be beneficial to use a product similar to bioactive glass, to enhance the proliferation of healthy cells and/or treat the infection.

### DEFINITIONS

The terms used in this application, if not otherwise defined, are those agreed on at the consensus conference on biomaterials in 1986, see Williams, DF (ed.): Definitions in biomaterials: Proceedings of a consensus conference of the European Society for Biomaterials, Chester, England. March 3-5, 1986. Elsevier, Amsterdam 1987, and Williams DF, Black J, Doherty PJ. Second consensus conference on definitions in biomaterials. In: Doherty PJ, Williams RL, Williams DF, Lee AJ (eds). Biomaterial-Tissue Interfaces. Amsterdam: Elsevier, 1992.

In this application, by bioactive component is meant a component that has been designed to elicit or modulate biological activity. By biocompatibility is meant the ability of a material used in a medical application to perform safely and adequately by causing an appropriate host response in a specific location.

The term particle refers to an entity of preferably irregular shape and having certain dimensions. The term particle size refers to the largest dimension of the particle.

In the present description, the abbreviation wt-% stands for weight percentage, and is typically expressed as a weight percentage of the total weight.

In this description, an extract means a solution obtained by immersing bioactive glass particles in water or saline to dissolve some of its components such as sodium, calcium, phosphate, and silica.

### OBJECTS AND SUMMARY OF THE INVENTION

It is an object of the present invention to provide a product that is suitable for use in the medical field, also in other forms than solid, and which has same benefits as bioactive glasses. Another object is to provide a product that accelerated the healing process of different wounds, such as diabetic wounds.

Another object of the present invention is to provide an aqueous solution for versatile application that can be integrated into various delivery systems, such as coatings for implants, dressings, or topical solutions.

The present invention is defined in the enclosed independent claims, with the dependent claims and this description describing some embodiments of the invention.

The present invention relates to a method for manufacturing an aqueous glass extract solution comprising 250-15000 mg/l of Na-ions, 5-300 mg/l of Ca-ions, 1-300 mg/l of P-ions and 30-1500 mg/l of Si-ions, the method comprising
- obtaining a bioactive glass composition comprising 44-65 weight-% of SiO₂, 5-26 weight-% of Na₂O, 0.5-25 weight-% of CaO and and 0.1-7 weight-% of P₂O₅, in the form of particles having a particle size of 1-1000 µm;
- adding the bioactive glass particles in water or saline solution, in an amount of 1-50 wt-%;
- extracting the ions into water or the saline solution by allowing the bioactive glass particles to dissolve for an extraction time of 1-120 h at a temperature of at least 20 °C; and
- recovering the supernatant aqueous glass extract solution.

The present invention relates also to an aqueous glass extract solution comprising 250-15000 mg/l of Na-ions, 5-300 mg/l of Ca-ions, 1-300 mg/l of P-ions and 30-1500 mg/l of Si-ions.

The present invention further relates to use of the aqueous glass extract solution in a medical application.

### DETAILED DESCRIPTION OF THE INVENTION

A typical method for manufacturing an aqueous glass extract solution comprising 250-15000 mg/l of Na-ions, 5-300 mg/l of Ca-ions, 1-300 mg/l of P-ions and 30-1500 mg/l of Si-ions, comprises
- obtaining a bioactive glass composition comprising 44-65 weight-% of SiO₂, 5-26 weight-% of Na₂O, 0.5-25 weight-% of CaO and 0.1-7 weight-% of P₂O₅, in the form of particles having a particle size of 1-1000 µm;
- adding the bioactive glass particles in water or saline solution, in an amount of 1-50 wt-%;
- extracting the ions into the solution by allowing the bioactive glass particles to dissolve for an extraction time of 1-120 h at a temperature of at least 20 °C; and
- recovering the supernatant aqueous glass extract solution.

The present invention thus provides a method for manufacturing an aqueous glass extract solution comprising certain amounts of ions. Most typically, the sodium ions are present in the form Na⁺, calcium ions in the form Ca²⁺, phosphate ions in the form PO₄³⁻ and silicon ions in the form Si⁴⁺. The amounts of the ions are determined typically using Inductively Coupled Plasma Optical Emission Spectroscopy, ICP-OES.

The present method thus provides an aqueous solution usable in various applications. Indeed, the solution can be integrated into different delivery systems, such as coatings for implants, dressings, or topical solutions.

The extract can also be infused into hydrogels, foam dressings, mixed with scaffolding materials, integrated into serums, creams and pastes, used to create gels and sprays, and used in injectable formulations.

The aqueous glass extract solution and its dried form (as described below) are believed to have at least some of the following properties: antibacterial, angiogenic, bone regenerative, soft tissue regenerative, nerve stimulation and action on harmful cells. Suitable application areas include wound treatment, wound lavage, prevention of dental biofilm formation, treatment of deep infections, nerve damages, dental infections, dermal infections, burn injuries, nasal infections, nail infections, eye infections, ear infections, lung infections, treatment of osteoporosis, treatment of hair loss, and action on tumour cells or tissues. As has been demonstrated below in the experimental section, the present glass extract solution may facilitate material containment within a wound, while presenting excellent biocompatibility, and having remarkable antibacterial properties, making them an effective alternative for managing wound healing.

The present glass extract solution can also be used in the form of a liquid (sol) that becomes a gel at a different temperature. For example, a hydrogel comprising the extract solution can be applied as a liquid (sol) below its gelation point to fill deep wounds, and it becomes a gel upon warming to body temperature. It is believed that at least for some combinations, such as when using a poloxamer together with the extract solution, an increased extract concentration may lower the hydrogel's gelation temperature due to the salt-out effect caused by the increased ionic species in the medium. The salting-out effect reduces poloxamer solubility, promoting earlier micelle formation and, therefore, gelation.

An advantage of using hydrogels comprising the extract is that they can both absorb fluids and release fluids. This is believed to be for wound healing, for example.

Different wounds and lesions have different healing times depending on i.a. their depth, width, moisture level and nature (or cause). For example, it has been found that infected diabetic foot wounds have an average healing time of 83 days. The different products comprising the present extract solution have different dissolution rates, which need to be taken into account in the planning of the treatment. For example, several applications at regular intervals may be needed during the wound-healing treatment. The frequency of reapplication depends on the wound characteristics (area, depth, moisture level...) and desired therapeutic outcome.

In the bioactive glass compositions, the weight-percentages are calculated of the final total weight of the glass.

The bioactive glass composition may further comprise 0-15 weight-% of K₂O, 0-6 weight-% of MgO and 0-4 weight-% of B₂O₃.

According to an embodiment, the composition of the bioactive glass is 45-54 weight-% of SiO₂, 22-25 weight-% of Na₂O, 19-25 weight-% of CaO and 3.5-6 weight-% of P₂O₅. According to another embodiment, the composition of the bioactive glass is 53 weight-% of SiO₂, 23 weight-% of Na₂O, 20 weight-% of CaO and 4 weight-% of P₂O₅. This glass is also known as S53P4-glass and sold under the trade name of BonAlive^{®}. A yet further preferred composition of bioactive glass is 45 weight-% of SiO₂, 24.5 weight-% of Na₂O, 24.5 weight-% of CaO and 6 weight-% of P₂O₅, also known as the Hench glass and sold under the trade name of e.g. NovaBone^{®} or GlassBone^{®}.

A mixture of different bioactive glasses can also be used, as long as the amounts of the components stay within the above limits. Further, the bioactive glass composition may be doped with therapeutically active ions, for example in a concentration of 0-5 wt-% of the total weight of the bioactive glass, such as 0-2 wt-%. The suitable concentration depends on the ion in question. Some possible ions are zinc (Zn), magnesium (Mg), silver (Ag), strontium (Sr), gallium (Ga), fluorine (F), iron (Fe), cobalt (Co), boron (B), lithium (Li), titanium (Ti), copper (Cu), barium (Ba), bismuth (Bi), chlorine (Cl), chromium (Cr), dysprosium (Dy), europium (Eu), gadolinium (Gd), ytterbium (Yb), thulium (Tm), germanium (Ge), gold (Au), holmium (Ho), iodine (I), lanthanum (La), manganese (Mn), molybdenum (Mo), nickel (Ni), niobium (Nb), nitrogen (N), palladium (Pd), rubidium (Rb), samarium (Sm), selenium (Se), tantalum (Ta), tellurium (Te), terbium (Tb), erbium (Er), tin (Sn), tungsten (W), vanadium (V), yttrium (Y) as well as zirconium (Zr).

The glass particles used in the method have a particle size of 1-1000 µm. The particle size may be for example less than 25 µm, i.e. 1-25 µm, or 25-500 µm.

The particle size can be measured by dynamic image analysis, for example with a CAMSIZER system, using ISO 13322-2:2021. Another option is to use sieve analysis. Any other suitable method can be used, and it is believed that all measurement methods lead to essentially same results and in case there are minor differences, the end result in the aqueous glass extract solution is not affected, since the particle size is only relevant for the extraction speed (the smaller the particles, the larger the total surface area).

The particle size can be for example from 1, 2, 3, 4, 5, 10, 15, 20, 15, 30, 40, 45, 50, 70, 90, 100, 120, 150, 200, 250, 300, 350, 400, 450, 500, 550, 600, 650, 700, 800, 850, or 900 µm up to 2, 3, 4, 5, 10, 15, 20, 15, 30, 40, 45, 50, 70, 90, 100, 120, 150, 200, 250, 300, 350, 400, 450, 500, 550, 600, 650, 700, 800, 850, 900, 950 or 1000 µm. Some typical particle size ranges are < 25 µm, < 45 µm, < 90 µm, < 120 µm, < 150 µm, < 250 µm, < 500 µm and < 1000 µm. It is also possible to combine different fractions, such as a fraction of 25-45 µm and a fraction of 90-500 µm. Typically, the smaller the particles, the better the yield of the extract solution, in terms of released ions.

The amount of the bioactive glass needed depends on the particle size, as the smaller the particle size, the smaller the amount needed, due to the large surface available in the small particles. For example, when using the particle size of less than 25 µm, a typical amount is 100 g of glass / litre of water or saline. When larger concentrations of glass were used, such as 250 g - 500 g / litre, the amounts of sodium and silicon ions were higher, but not the amounts of calcium and phosphate ions.

Typically, the larger the particle size, the higher the concentration of the glass and/or the dissolution time needed.

The aqueous solution is prepared in water or saline as solvent, as these are usable in medical applications. Water does in principle not have any ions, while saline is a solution of 0.9 % NaCl in water, i.e. 9 g NaCl/litre, thus the content of sodium in saline is 3600 mg/litre. Therefore, when saline is used as the solvent, the amount of sodium in the aqueous glass extract solution may be higher than if water is used, in particular is the same amount and same particle size of the bioactive glass is used.

A typical aqueous glass extract solution comprises 250-15000 mg/l of Na-ions, 5-300 mg/l of Ca-ions, 1-300 mg/l of P-ions and 30-1500 mg/l of Si-ions.

According to an embodiment, the solvent is water and the amount of Na-ions is 250-10000 mg/l. According to another embodiment, the solvent is saline and the amount of Na-ions is 3600-15000 mg/l. The other ions are present in the same amount irrespective of the solvent.

Most typically, the aqueous glass extract solution is obtainable by the method described above.

The bioactive glass particles are used in an amount of 1-50 wt-% based on the total weight of the particles and water or saline. According to an embodiment, the amount of bioactive glass particles is 5-10 wt-%. This amount is particularly preferably, when the particle size is less than 25 µm. The amount of the bioactive glass particles can be from 1, 2, 3, 4, 5, 7, 10, 15, 20, 25, 30, 34, 40 or 45 wt-% up to 2, 3, 4, 5, 7, 10, 15, 20, 25, 30, 34, 40, 45 or 50 wt-%.

The bioactive glass particles are allowed to dissolve in water or saline, i.e. an extraction of the ions to the solution takes place. After the desired time, the supernatant aqueous glass extract solution is recovered. In case all the bioactive glass has dissolved, it can all be recovered.

The bioactive glass particles are extracted, i.e. allowed to dissolve, for an extraction time of 1-120 h. According to an embodiment, this extraction time is 2-8 h. The time can be for example from 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 105, 110 or 115 hours up to 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 105, 110, 115 or 120 hours.

Extraction is carried out at a temperature of 20 °C or more. For example, an autoclave can be used, and the temperature can be for example that used for sterilisation, such as 121 °C or 132 °C. The extraction can be enhanced by applying constant or periodic shaking during the extraction.

Phosphate together with calcium starts to precipitate on the surface of the glass before 24 hours, by which time the level of phosphate in the solution may be low. One optimal extraction time is 2-8 hours.

According to an embodiment, the method further comprises
- obtaining a first supernatant aqueous glass extract solution after a first extraction time;
- obtaining a second supernatant aqueous glass extract solution after a second extraction time, which second extraction time is longer than the first extraction time; and
- mixing the first and second supernatant aqueous glass extract solutions.

According to one example, the first supernatant aqueous glass extract solution is obtained after an extraction time of 1-8 hours, and the second supernatant aqueous glass extract solution is obtained after 24-120 hours of extraction time, both from the same reactor. These can then be mixed in a ratio of 1:4 - 4:1.

It is also possible to combine two or more extracts from different reactors, i.e. using additionally different amounts and/or particle sizes for the bioactive glass.

An advantage of combining two or more extracts is that it may allow obtaining higher amounts of sodium and silicon ions, while also keeping the amount of calcium and phosphate ions at a high level (since they no longer can precipitate back to the surface of the glass once the glass has been removed).

The pH of the solution rapidly raises when the bioactive glass is added to the solvent, and remains at 11-12 in a non-buffered solution, irrespective of the extraction time. The concentration of the ions in the solution raises with time, with the above-mentioned exception of phosphate and calcium which decrease over time due to calcium phosphate precipitation on the glass surface.

The amount of ions in the extract may vary significantly depending on whether a buffer is used, and which buffer is used, as the concentration of Na, Ca, P and Si ions in the original solvent influences on the amount of ions that are extracted.

The present invention further relates to use of the aqueous glass extract solution in a medical application.

The medical application may be selected from a group consisting of dentistry, treatment of an infection, treatment of a wound, treatment of osteoporosis and repair of nerve damages. Suitable application areas include wound treatment, wound lavage, prevention of dental biofilm formation, treatment of deep infections, nerve damages, dental infections, dermal infections, burn injuries, nasal infections, nail infections (such as nail fungus), eye infections, ear infections, lung infections, implant coating, treatment of osteoporosis and action on tumour cells or tissues. The wound may be for example an infected wound (acute or chronic), a burn wound, a surgical wound or a scarred wound.

The extract may be used as such, diluted with water or saline, mixed with another solution, or in the form of a gel, an emulsion, a cream, a foam, a spray, or a paste. It may also be dried and used in dried form, as explained in more detail below.

The extract has a basic pH and therefore all materials prepared from it must withstand high pH values, and remain stable for its composition, safety and performance, for a desired period (shelf life). While it is possible to adjust the pH of the solution closer to neutral pH, this would mean that a part of the dissolved silicon precipitates. Not all polymers and hydrogels for example can withstand such basic pH, and thus the pH needs to be kept in mind when manufacturing the material comprising the extract. Recent studies show that slightly alkaline pH could be beneficial for wound healing.

When a hydrogel is used, the glass extract solution is typically mixed with a gelling agent. The conditions for the mixing may vary depending on the gelling agent. The conditions may include for example temperature, mixing time and mixing speed and means.

According to an embodiment, the extract is used in the form of a gel, with a poloxamer (thermoreversible gelation), a modified chitosan (for example carboxymethylated), agarose, alginate, a polyurethane hydrogel, polyvinyl alcohol (PVA), polyethylene glycol (PEG), polyethylene glycol-based hydrogel, polyacrylamide, a carbomer, poly(N-isopropylacrylamide) or a mixture thereof.

According to an embodiment, the extract is used in a dried form. The extract solution may be dried by evaporation, to provide a powder, or the extract may be impregnated into a porous material followed by evaporation. The porous material may be for example a sponge, a cloth or a scaffold.

Water can be evaporated to the point that the ions supersaturate and precipitate to form a mixture of amorphous or crystalline solid deposits. These deposits can be ground to a fine powder to form a homogeneous powder. This powder easily and rapidly dissolves into an aqueous solution. The powder can be stored and used later as a starting material for producing an identical aqueous solution by adding an equal amount of water as was initially there before the evaporation. Adding less than the initial amount of water will produce a more concentrated solution while adding more than the initial amount of water will produce a diluted solution, respectively. The powder may also have a longer shelf life than the extract solution.

The dried extract may also be used for manufacturing a non-aqueous putty, a non-aqueous paste, a scaffold, or a composite, such as dental composite. The dried extract may also be used in various resins and gums. The dried extract may specifically can be used for various types of solid formulations and composites. Alternatively, the powder can be compressed into pellets or beads before mixing.

In case the dried extract is manufactured to a scaffold, or the liquid is first impregnated into a scaffold and then dried, the scaffold may be made of (biodegradable) polymers, ceramics and/or bioactive glass.

Both the extract and its dried form may be used in 3D printing to form 3D structures that are used for implantation. For example, the gels mentioned above can be used in 3D printing.

The extract may be used together with an active agent selected from a group consisting of an antibiotic, an anti-inflammatory agent, a growth factor, an anticancer agent, an antimicrobial agent, an anti-osteoporotic agent, a stem cell, a peptide, a protein, and mixtures thereof.

### EXPERIMENTAL PART

The aim of this experimental part was to evaluate the regenerative and/or antimicrobial performance of the extract and extract-based materials on defects and infections of the human body.

### Materials and methods

### Bioactive glass

Bioactive glass S53P4, with a wt-% composition of 53 % SiO₂, 23 % NaO, 20 % CaO and 4 % P₂O₅ was made from mixtures of Belgian sand for SiO₂ and analytical grades of Na₂CO₃, CaCO₃ and CaHPO₄.2H₂O. The glass was melted in a platinum crucible for 3 hours at 1360 °C. After casting in a preheated graphite mould, the glass was crushed and remelted for homogenisation. Annealed glass blocks were crushed and sieved to different size fraction ranges.

The main fraction that was used for the preparation of the extract at different concentrations was the fraction with a particle size of less than 25 µm.

### Preparation of the extract and results

### Treatment of leg wounds

Bioactive glass extract of a specific concentration was prepared using S53P4 bioactive glass powder having a particle fraction size < 25 µm. The powder was measured in a 50 ml plastic Falcon tube to the 5 ml mark and water was added to the 50 ml mark. The tube was closed tightly, and the content was mixed by shaking and turning the tube upside down for about 15 seconds in room temperature. The shaking and turning upside down were repeated every ten minutes during the first hour after which it was repeated every hour. After six hours the tube was left untouched for three hours during which time the powder became sedimented at the bottom of the tube. At the nine-hour time point the extract was decanted to a fresh 50 ml Falcon tube without disturbing the sediment.

The extract was used for wetting properly a waterproof Leukoplast^{®} Leukomed T plus dressing. The dressing was then carefully but tightly placed onto a one-day old leg wound and made sure the extract did not escape the dressing. The dressing was replaced daily with a new dressing which had been wetted properly with the extract. In seven days, the wound was closed, and the skin appeared normal to the eye. In similar but smaller untreated wounds a scab appeared on the wound and the healing of the wound took 3-4 weeks. No infection in any of the aforementioned wounds was observed.

### Treating nasal infections

The extract used for the spray was prepared in the following manner: 50 g of S53P4 powder (< 42 µm) was weighed into a glass bottle and then filled to 0.5 litre with saline. The bottle was closed tightly, and the content was properly mixed by turning the bottle upside down in room temperature. The mixing was repeated every 30 minutes over the course of 8 hours and the bottle was left standing overnight. After 24 hours of the first mixing the clear solution was filtered through a membrane having a pore size of 0,45 µm. A sufficient portion of the filtered extract was poured into a spray bottle, ready to be applied.

During a Covid-19 infection, a patient lost completely the sense of taste and smell at an early stage of the illness, and the following treatment was started soon after. A house-hold spray bottle was used to spray pure extract directly into the upper part of the nasal canal to make sure it reached the sensory cells. Likewise, extract was sprayed onto the tongue to reach the taste buds. The following spraying frequency was used: four sessions, each lasting one hour, were spread throughout the day. During each one-hour session, the extract was sprayed to both nostrils and tongue at an interval of 15 minutes, thus, four spray events in one hour. After 24 hours, the taste and smell senses had fully recovered, and the treatment ended. During the course of the illness, the senses started to fade away two more times and repeating the spray treatment gave full and fast recovery both times. According to literature, in average, Covid-19 causes the loss of these senses for several weeks.

### Treating toenail infections

The extract used for the toenail treatment was prepared in the following manner.

Day 1: 100 g of S53P4 powder (< 25 µm) was weighed into a glass bottle, 50 ml of PEG 400 was added, and then filled to 1 litre with warm water (55 °C). The role of PEG 400 was to make the extract more permeable to the toenail. The bottle was closed tightly, and the content was properly mixed by turning the bottle upside down in room temperature. The mixing was repeated every 30 minutes over the course of 8 hours and the bottle was left standing for 24 hours.

Days 2-3: After 24 hours the mixing was continued as before, and the mixing was performed four times during day 2 and day 3. Between each mixing, the bottle was left standing.

Day 4: The clear solution was decanted to a new bottle and the sedimented powder was left behind. From the new bottle, part of the extract was poured into a container.

A 82-year old patient with severe long-lasting toenail fungal infection on all ten toes was treated with S53P4 extract by dipping the toes of the right foot into extract in the container and keeping the toes immersed for one hour each day. The treatment lasted for about two months, during which time, in about three weeks, the emerging newly grown nail appeared visually healthy, and over time the normal-looking nail kept on expanding at the rate of the growth of the nail. After two months, the healthy-looking nail covered one-fourth of the nail in the nail having the fastest growth rate. The nail of the big toe behaved differently from the other nails since a longitudinal part of the nail was missing. During the treatment, new nail formation appeared directly from the middle of the nail bed without connection to the nail root from where new nails normally grow, thus showing induction of new nail growth. After the two-month treatment period the treatment needed to be terminated and the full recovery to infection-free nails was not observed, however, the initial results were promising and showed a clear impact the S53P4 extract on fungus-infected nail.

### Treating dermal infections

The extract used was prepared in the following manner: 150 g of S53P4 particles (25-500 µm) was weighed into a glass bottle and the bottle was filled with boiling water to 1 litre. The bottle was closed tightly, and the content was mixed by shaking and turning the bottle upside down 20 times. The same mixing procedure was repeated over the course of the next 24 hours ten more times.

A chronic case of tinea cruris was treated by soaking hand towels in the extract and the towels were placed to cover the affected area and kept in place overnight every day for a one-week period. No other treatment was used. After one week the skin appeared normal and the itching and redness had disappeared. The symptoms have not reappeared even after several months of the treatment.

An acute infection in a folded skin area was treated by placing a part of a hand towel, which had been soaked in extract, between the folded skin and kept in place for 24 hours. The infection disappeared together with the itchiness and redness and the skin area has stayed without any signs of the infection recurring for several months.

### Prevention of dental biofilm formation

The extract for this experiment was prepared by weighing 20 g of < 25 µm S53P4 powder into a glass bottle and filling to 200 ml. After mixing by turning the bottle upside down 20 times the bottle and its content was autoclaved at 121 °C for 30 minutes. Once the bottle and its contents cooled down the extract was filtered by using a 0.45 µm filter

After washing of teeth, a small piece of hand paper was soaked with the extract and placed on two lower left incisors to cover the entire front side of the teeth. The same was done for the lower right incisors with paper soaked only in water. The papers were kept in place for 15 minutes. Throughout the day, five similar procedures were implemented each two hours. Visual observation showed a clear difference in the level of plaque formation between the teeth, the teeth treated with extract having much lower visible plaque formation at the end of the day at twelve hours.

## Claims

1. A method for manufacturing an aqueous glass extract solution comprising 250-15000 mg/l of Na-ions, 5-300 mg/l of Ca-ions, 1-300 mg/l of P-ions and 30-1500 mg/l of Si-ions, the method comprising
- obtaining a bioactive glass composition comprising 44-65 weight-% of SiO₂, 5-26 weight-% of Na₂O, 0.5-25 weight-% of CaO and 0.1-7 weight-% of P₂O₅, in the form of particles having a particle size of 1-1000 µm;
- adding the bioactive glass particles in water or saline solution, in an amount of 1-50 wt-%;
- extracting the ions into water or the saline solution by allowing the bioactive glass particles to dissolve for an extraction time of 1-120 h at a temperature of at least 20 °C; and
- recovering the supernatant aqueous glass extract solution.

2. The method according to claim 1, wherein the bioactive glass composition further comprises 0-15 weight-% of K₂O, 0-6 weight-% of MgO and 0-4 weight-% of B₂O₃.

3. The method according to claim 1 or 2, wherein the composition of the bioactive glass is 45-54 weight-% of SiO₂, 22-25 weight-% of Na₂O, 19-25 weight-% of CaO and 3.5-6 weight-% of P₂O₅.

4. The method according to any one of the preceding claims, wherein the extraction time is 2-8 h.

5. The method according to any one of the preceding claims, further comprising
- obtaining a first supernatant aqueous glass extract solution after a first extraction time;
- obtaining a second supernatant aqueous glass extract solution after a second extraction time, which second extraction time is longer than the first extraction time; and
- mixing the first and second supernatant aqueous glass extract solutions.

6. The method according to any one of the preceding claims, wherein the amount of bioactive glass particles is 5-10 wt-%.

7. The method according to any one of the preceding claims, wherein the particle size of the bioactive glass particles is 1-25 µm.

8. An aqueous glass extract solution comprising 250-15000 mg/l of Na-ions, 5-300 mg/l of Ca-ions, 1-300 mg/l of P-ions and 30-1500 mg/l of Si-ions.

9. The aqueous glass extract according to claim 8, wherein the solvent is water and the amount of Na-ions is 250-10000 mg/l.

10. The aqueous glass extract according to claim 8, wherein the solvent is saline and the amount of Na-ions is 3600-15000 mg/l.

11. Use of an aqueous glass extract solution according to any one of the claims 8-10 in a medical application.

12. Use according to claim 11, wherein the medical application is selected from a group consisting of dentistry, treatment of an infection, treatment of a wound, treatment of hair loss, treatment of osteoporosis, action on tumour cells or tissues and repair of nerve damages.

13. Use according to claim 11 or 12, wherein the extract is used as such, mixed with another solution, or in the form of a gel, a film, an emulsion, a cream, a foam, a spray, a paste, in dried form, or any combination thereof.

14. Use according to claim 13, wherein the extract is used in the form of a gel, using a poloxamer, a modified chitosan, agarose, alginate, a polyurethane hydrogel, polyvinyl alcohol, polyethylene glycol, polyethylene glycol-based hydrogel, polyacrylamide, a carbomer, poly(N-isopropylacrylamide), or a mixture thereof.

15. Use according to claim 13, wherein the extract is used in dried form for manufacturing a non-aqueous putty, a non-aqueous paste, a scaffold, or a composite.
